## Europäisches Patentamt
## European Patent Office
## Office européen des brevets

(19)

(11) Veröffentlichungsnummer: **0 105 443**
**B1**

(12) # EUROPÄISCHE PATENTSCHRIFT

(45) Veröffentlichungstag der Patentschrift:
02.07.86

(51) Int. Cl.⁴: **C 12 Q 1/00,** C 12 Q 1/28,
C 12 Q 1/32

(21) Anmeldenummer: 83109587.2

(22) Anmeldetag: 27.09.83

(54) **Verfahren zur selektiven Herstellung reduzierter Sauerstoffspezies und hierfür geeignete Reagentien.**

(30) Priorität: 01.10.82 DE 3236388

(43) Veröffentlichungstag der Anmeldung:
18.04.84 Patentblatt 84/16

(45) Bekanntmachung des Hinweises auf die Patenterteilung:
02.07.86 Patentblatt 86/27

(84) Benannte Vertragsstaaten:
AT BE CH DE FR GB IT LI LU NL SE

(73) Patentinhaber: Boehringer Mannheim GmbH,
Sandhofer Strasse 116, D-6800 Mannheim 31 (DE)

(72) Erfinder: Elstner, Erich, Prof. Dr. rer. nat.,
Wildmoosstrasse 18, D-8038 Gröbenzell (DE)

(56) Entgegenhaltungen:
EP-A-0 007 058
EP-A-0 016 845
DE-A-2 737 290
DE-A-3 106 601

CLINICAL CHEMISTRY, Band 27, Nr. 10, Oktober 1981, Seiten 1686-1689, Easton, USA, F.J. GELLA et al.: "Colorimetry of diaphorase in commercial preparations and clinical chemical reagents by use of tetrazolium salts"

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der enzymatischen Reduktion des Sauerstoffmoleküls entstehen je nach Anzahl der übertragenen Elektronen verschiedene, zum Teil sehr reaktive Produkte. Die folgende Tabelle gibt einen Überblick über die möglichen Redox-Reaktionen. Ferner werden Beispiele für Enzyme angegeben, die die jeweilige Redox-Reaktion zu katalysieren vermögen.

Tab. 1: Redox-Reaktionen des Sauerstoffmoleküls

| Nr. | Anzahl der übertra-genen Elektronen | Produkt | Enzymbeispiel |
|-----|-------------------------------------|---------|---------------|
| 1 | 1 E + 2 E | $O_2^{\cdot-}$; $H_2O_2$ | Xanthinoxidase |
| 2 | 2 E | $H_2O_2$ | Glucoseoxidase |
| 3 | 3 E | OH.-Radikal | (Unspezifität der Xanthinoxidase |
| 4 | 4 E | $H_2O$ | Cytochromoxidase |

Insbesondere das bei einem Ein-Elektronenschritt entstehende Superoxid ($O_2^-$) hat im menschlichen und tierischen Organismus bei der Abwehr von Fremdstoffen eine erhebliche Bedeutung. Seit der Entdeckung der Superoxiddismutasen, Enzyme, die die Dismutation von Superoxid in Wasserstoffperoxid und Sauerstoff katalysieren, im Jahre 1969 hat das Gebiet der Sauerstoffbiochemie erheblich an wissenschaftlichem wie auch wirtschaftlichem Interesse gewonnen. Fragen, welche Reduktionsprodukte bei den verschiedensten biochemischen Sauerstoffreduktionen entstehen, wie diese Prozesse durch die beteiligten Reaktionspartner beeinflußt werden, welche Substrate benötigt werden, welche Zwischenprodukte entstehen usw., stehen dabei im Vordergrund. Superoxiddismutase wird bereits als Therapeutikum hauptsächlich gegen Gelenkentzündungen eingesetzt. Eine zuverlässige Bestimmungsmethode ist für die Dosierung von wirtschaftlicher Bedeutung.

Bei dieser Fragestellung stellt sich immer wieder das Problem, die verschiedensten Sauerstoffreduktionsprodukte zu analysieren sowie die Konzentration der an den Reduktionsprozessen beteiligten Enzyme und Substrate zu bestimmen. Hierfür gibt es eine Reihe von Bestimmungsverfahren, insbesondere für das Superoxid, das Wasserstoffperoxid, das Hydroxyl-Radikal sowie die Superoxiddismutasen. Nachteilig an diesen Bestimmungsverfahren ist es bisher, daß die bekannten enzymatischen Sauerstoff-Reduktionsprozesse stets zu einem Gemisch verschiedener Reduktionsprodukte führen, wodurch definitive Aussagen über die physiologische Wirkung der Reduktionsprodukte sowie die Anwendung der Reduktionsprodukte zur Analytik sie umsetzender Enzyme und an ihrer Bildung beteiligter Reaktionspartner entscheidend beeinträchtigt werden. So liefert das bei der heutigen Superoxid- bzw. Superoxiddismutase-Analytik hauptsächlich eingesetzte Superoxid-generierende System, die Xanthinoxidase, nicht nur Superoxid, sondern auch Wasserstoffperoxid (vgl. Tab. 1, Nr. 1) und zum Teil auch das sehr reaktive Hydroxyl-Radikal.

Aufgabe der vorliegenden Erfindung war es deshalb, ein einfaches Verfahren bereitzustellen, mit dem es möglich ist, selektiv in einem Ein-, Zwei- bzw. Drei-Elektronenschritt Superoxid, Wasserstoffperoxid bzw. Hydroxyl-Radikale zu erzeugen. Oberraschenderweise läßt sich diese Aufgabe dadurch lösen, daß Sauerstoff mit NAD(P)H in Gegenwart einer NAD(P)H-abhängigen, nicht autoxidablen Diaphorase und eines speziell ausgewählten autoxidablen Redoxpartners reduziert wird.

Gegenstand der Erfindung ist demgemäß ein Verfahren zur selektiven Herstellung der reduzierten Sauerstoffspezies Superoxid, Wasserstoffperoxid und Hydroxyl-Radikal, dadurch gekennzeichnet, daß Sauerstoff mit NAD(P)H in Gegenwart einer NAD(P)H-abhängigen, nicht-autoxidablen Diaphorase und eines speziell ausgewählten autoxidablen Redoxpartners reduziert wird.

NAD(P)-abhängige, nicht-autoxidable Diaphorasen sind Diaphorasen, welche die Reduktion des Sauerstoffs durch NAD(P)H nicht zu katalysieren vermögen.

Im Sinne der vorliegenden Erfindung sind prinzipiell alle derartigen Diaphorasen brauchbar. Besonders vorteilhaft wird eine Diaphorase-aktive NADP-Ferredoxin-(Cyt c)-Oxidoreduktase (E.C.1.6.99.4) eingesetzt, die beispielsweise aus Spinat oder Euglena gracilis gewonnen werden kann. Sehr gut brauchbar sind auch die handelsüblichen Diaphorase aus Clostridium kluyveri sowie eine Diaphorase aus Mikroorganismen, die von der Firma Boehringer Mannheim GmbH vertrieben wird.

Als autoxidable Redoxpartner kommen alle möglichen Stoffe in Frage, die fähig sind, ein oder mehrere Elektronen auf Sauerstoff zu übertragen. Für den Ein-Elektronenschritt eignen sich solche Redoxpartner, deren Ein-Elektronen-Redoxpotential $E'_0$ in dem Bereich von -150 mV bis -500 mV liegt. Insbesondere haben sich Bipyridilium-Salze, beispielsweise 1,1'-Dimethyl-4,4'-bipyridiliumchlorid (Paraquat) und 1,1'-Dimethylen-2,2'-bipyridilium-chlorid (Diquat), Triazoniumsalze, beispielsweise 1-Methyl-4(4,6-dimethyl-2-symtriazinyl)-pyridinium-bromid und 1-Methyl-4-(2-symtriazinyl)-pyridinium-bromid, Anthrachinon-Derivate, beispielsweise Anthrachinon-2-sulfonsäure, Nitrofuran-Derivate, beispielsweise Nitrofurantoin, Ferredoxine, Pteridine und Isoalloxazine, beispielsweise Riboflavin im Rahmen der Erfindung als brauchbar erwiesen.

Als Redoxpartner für den Zwei-Elektronenschritt können solche Substanzen eingesetzt werden, deren Zwei-Elektronen-Redoxpotential $E'_0$ im Bereich von 0 bis 150 mV liegt. Als solche sind vor allem Chinon-Derivate, insbesondere p-Benzochinon-Derivate, wie 2,5-Dibromo-3-methyl-6-isopropyl-p-benzochinon und 2,3-Dimethyl-5,6-methylendioxy-p-benzochinon, zu nennen.

Zur Herstellung von Hydroxyl-Radikalen, dem Endprodukt des Drei-Elektronenschritts, wird die NAD(P)H-abhängige, nichtautoxidable Diaphorase mit solchen Substanzen gekoppelt, die im reduzierten Zustand in luftgesättigter Lösung ein Elektron auf Wasserstoffperoxid übertragen können. Als ganz besonders geeignet in diesem Sinne sind Anthrachinon-Derivate, wie Anthrachinon-2-sulfonsäure, Nitrofurantoin und Ferredoxine zu nennen.

Die Ein- oder Zwei-Elektronen-Reduktionen des Sauerstoffs wird vorzugsweise in Luft- bzw. Sauerstoffgesättigter Lösung durchgeführt. Die Temperatur kann in relativ weiten Grenzen variiert werden. Vorzugsweise wird bei 18 - 25°C oder bei Raumtemperatur gearbeitet. Es können aber auch niedrigere oder höhere Temperaturen gewählt werden. Nach oben wird der Temperaturbereich durch die Stabilität des gewählten Enzyms begrenzt. Gegebenenfalls wird dem Reagenzgemisch ein geeigneter Puffer zugesetzt. Im Rahmen der Erfindung können alle puffer verwendet werden, die im pH-Bereich von 6 - 9 wirksam sind und die keine Radikalfänger darstellen. Bewährt hat sich besonders ein Phosphatpuffer mit pH 7 bis 8. Der Puffer wird vorteilhaft in einer Konzentration von 1 - 500 vorzugsweise 50 - 200 mmolar eingesetzt.

Zur selektiven Erzeugung der Hydroxyl-Radikale wird unter anaeroben Bedingungen der Reaktionslösung aus der NAD(P)H-abhängigen, nicht-autoxidablen Diaphorase, dem autoxidablen Redoxpartner und ggf. dem geeigneten Puffersystem in einem geeigneten Lösungsmittel, vorzugsweise Wasser, Wasserstoffperoxid zugesetzt. Es kann auch unter aeroben Bedingungen und Superoxiddismutase-Zusatz gearbeitet werden. Dabei wird das primär entstehende Superoxid rasch zu Sauerstoff und Wasserstoffperoxid dismutiert. Letzteres wird dann in einem weiteren Ein-Elektronenschritt selektiv zum Hydroxyl-Radikal reduziert.

Ein weiterer Gegenstand der Erfindung ist ein Reagenz zur Durchführung des erfindungsgemäßen Verfahrens, das gegebenenfalls in getrennten Einheiten in Form einer Lösung, eines Pulvergemisches, einer Reagenztablette oder eines Lyophilisats NAD(P)H, die NAD(P)H-abhängige, nicht-autoxidable Diaphorase, den autoxidablen Redoxpartner sowie gegebenenfalls ein geeignetes Puffersystem enthält.

Nach dem erfindungsgemäßen Verfahren bzw. mit Hilfe des erfindungsgemäßen Reagenzes ist es somit möglich, das Sauerstoffmolekül selektiv zu ganz bestimmten Reduktionsprodukten zu reduzieren. Ohne störende Nebeneffekte befürchten zu müssen, kann daher die Wirkung der einzelnen Sauerstoffreduktionsprodukte auf verschiedene Substanzen, enzymatische Systeme usw. beobachtet werden. Damit können Fragestellungen zur differentiellen Toxizität der einzelnen Sauerstoffreduktionsprodukte bzw. zur Sensitivität verschiedener biologischer Materialien gegenüber den Sauerstoffreduktionsprodukten selektiv untersucht werden. Hierzu werden die zu untersuchenden Proben mit einem Testgemisch versetzt, das neben NAD(P)H eine NAD(P)H-abhängige, nicht-autoxidable Diaphorase und einen autoxidablen Redoxpartner zur Erzeugung von Superoxid, Wasserstoffperoxid oder einem Hydroxylradikal sowie gegebenenfalls ein geeignetes Puffersystem enthält. Der Effekt, der von dem erzeugten Sauerstoffreduktionsprodukt bewirkt wird, kann nach üblichen, dem Fachmann bekannten Methoden analysiert werden.

Im Prinzip ist es auf diese Weise möglich, die verschiedensten Reaktionspartner in solchen Reaktionen zu messen, bei denen die reduzierten Sauerstoffspezies als ein Reaktionspartner auftreten oder die durch die reduzierten Sauerstoffspezies induziert bzw. katalysiert werden. Hier sei beispielsweise die Oxidation von Hydroxylamin zu Nitritionen durch Superoxid genannt. Da diese Oxidationsreaktion stöchiometrisch verläuft, kann das erfindungsgemäße Superoxid-liefernde System zur Bestimmung von Hydroxylamin benutzt werden. Die bei der Oxidation von Hydroxylamin entstehenden Nitritionen werden hierzu nach üblichen Methoden bestimmt.

Verschiedene Antibiotika, Drogen, wie z.B. Adriamycin, Bleomycin, Nitrofurantoin und Xenobiotika, wie z.B. Herbizide und Insektizide, sind potentielle autoxidable Redoxpartner für eine Ein-, Zwei- oder Drei-Elektronen-Reduktion des Sauerstoffs nach dem erfindungsgemäßen Prinzip. Es ist mit Hilfe des erfindungsgemäßen Verfahrens möglich, die Wirkung einer bestimmten Droge bzw. eines bestimmten Antibiotikums auf die Sauerstoffreduktion zu untersuchen. Hierzu wird ein Testsystem, das NAD(P)H und eine NAD(P)H-abhängige, nicht-autoxidable Diaphorase und die zu untersuchende Substanz enthält, mit Sauerstoff in Kontakt gebracht. Das entstehende Sauerstoffreduktionsprodukt wird nach den üblichen Verfahren bestimmt. Beispielsweise kann mit Hilfe von Hydroxylamin geprüft werden, ob Superoxid entstanden ist. Eventuell durch Oxidation des Hydroxylamins erhaltene Nitritionen können nach den üblichen Methoden gemessen werden.

Zum Nachweis von eventuell entstandenem Wasserstoffperoxid stehen dem Fachmann zahlreiche bekannte Bestimmungsmethoden zur Verfügung. Speziell geeignet erwies sich dafür die in vielen Varianten bekannte Reaktion nach TRINDER.

Auf möglicherweise gebildete Hydroxyl-Radikale kann die Testlösung beispielsweise mit Hilfe von Methionin untersucht werden. Das sich bei diesem bekannten Test bildende Ethylen wird gaschromatographisch bestimmt. Die Möglichkeit, in einem Ein-Elektronenschritt mit einer NAD(P)H-abhängigen, nicht-autoxidablen Diaphorase und einem geeigneten autoxidablen Redoxpartner selektiv Superoxid zu erzeugen, führt auch zu einem selektiveren Superoxiddismutase-Nachweis. Superoxiddismutase (SOD) katalysiert die folgende

Reaktion: $2O_2^- + 2H^+ \rightleftharpoons H_2O_2 + O_2$

Superoxiddismutase-Bestimmungen beruhen im allgemeinen auf folgendem Prinzip: Eine Superoxid-liefernde Reaktion wird mit zwei Superoxid-verbrauchenden Reaktionen gekoppelt, wo bei eine der Superoxid-verbrauchenden Reaktion eine Indikatorreaktion und die andere Superoxid-verbrauchende Reaktion die Superoxiddismutase-Reaktion darstellt. Die Superoxiddismutase-Reaktion konkurriert folglich mit der Indikatorreaktion um die vorhandenen Superoxid-Moleküle. Je mehr Superoxiddismutase folglich in der Testlösung vorliegt, desto stärker wird die Indikatorreaktion gehemmt. Eine Obersicht über die augenblicklich gebräuchlichsten Superoxiddismutase-Bestimmungen ist dem Artikel "Superoxiddismutase-Assays: A Review of Methodology aus "Superoxide und Superoxide-Dismutases', A.M. Michelson, J.M. McCord, I. Fridovich, Hrsg., Academic Press New York, 1977", zu entnehmen.

Als Superoxid-liefernde Reaktion wird bei den augenblicklich am meisten verwendeten Superoxiddismutase-Tests die Xanthinoxidase-Reaktion verwendet. Wie bereits erwähnt, liefert die Xanthinoxidase-Reaktion nicht spezifisch Superoxid, sondern die Nebenreaktionen wird auch Wasserstoffperoxid und das Hydroxylradikal gebildet. Dadurch wird die Superoxiddismutase-Bestimmung erheblich gestört. Als Indikatorreaktion werden üblicherweise der Cytochrom-Reduktionstest, der sog. Adrenalintest oder der Nitro-Blau-Tetrazolium-Test benutzt. Diese Vielkomponentensysteme bringen ebenfalls erhebliche Schwierigkeiten mit sich, wenn gleichzeitig verschiedene reduzierte Sauerstoffspezies auftreten. Ferner ist bei den Vielkomponentensystemen mit ungewollten, aber stets meßbaren Zusatzkatalysen durch unspezifisch gebundene Obergangsmetallionen, beispielsweise Eisen und Kupfer, zu rechnen.

Wird in erfindungsgemäßer Weise erzeugtes Superoxid zur Bestimmung der Superoxiddismutase eingesetzt, so wird ein störungsfreier Test erhalten. Als Indikatorreaktion kann jede bekannte Indikatorreaktion gekoppelt werden. Besonders vorteilhaft hat sich zur Bestimmung der Superoxiddismutase die Kombination der erfindungsgemäßen Superoxid-liefernden Reaktion mit dem Hydroxylamin-Test erwiesen. Hierbei wird Hydroxylamin durch das entstehende Superoxid zu Nitrition oxidiert, das nach Diazotieren und nachfolgender Azokupplung photometrisch gemessen werden kann.

Ein weiterer Gegenstand der Erfindung ist ein Verfahren zur Bestimmung von Superoxiddismutase durch Koppeln einer Superoxid-liefernden Reaktion mit einer Superoxid-verbrauchenden Indikatorreaktion, die mit der durch Superoxiddismutase katalysierten Dismutation des Superoxids konkurriert, wobei als Superoxid-liefernde Reaktion die Reduktion des Sauerstoffs durch NAD(P)H in Gegenwart einer NAD(P)H-abhängigen, nichtautoxidablen Diaphorase, eines geeigneten autoxidablen Ein-Elektronen-Redoxpartners sowie gegebenenfalls eines geeigneten Puffersystems verwendet wird.

Gegenstand der Erfindung ist ferner ein Reagenz zur Bestimmung der Superoxiddismutase, das in Form einer Lösung, eines Pulvergemischs, einer Reagenztablette oder eines Lyophilisates NAD(P)H, eine NAD(P)H-abhängige, nicht-autoxidable Diaphorase, einen autoxidablen Ein-Elektronen Redoxpartner, die Komponenten für eine geeignete Indikatorreaktion sowie gegebenenfalls ein geeignetes Puffersystem enthält.

Als NAD(P)H-abhängige, nicht autoxidable Diaphorasen sind besonders die bereits auf Seite 3 genannten Enzyme geeignet. Als autoxidable Ein-Elektronen-Redoxpartner sind die auf Seite 3 aufgezählten Substanzen bevorzugt. Als Indikatorreaktion wird vorzugsweise die Oxidation des Hydroxylamins zu Nitrit-Ionen mit nachfolgender Bestimmung der Nitrit-Ionen benutzt. Als Komponenten dieser Reaktion werden dann dem vorstehend genannten Reagenz Hydroxylamin und die zur Bestimmung der Nitrit-Ionen erforderlichen Substanzen, z.B. Sulfanilsäure und α-Naphthylamin oder Sulfanilamid und Naphthylethylendiamin, zugesetzt.

Die verschiedenen Bestandteile sind vorzugsweise in solchen Mengenverhältnissen im Reagenz enthalten, daß in 2 ml fertiger Testlösung die folgenden Konzentrationsverhältnisse vorhanden sind:

1 - 10 µmol NAD(P)H
0,05 - 0,5 mg Diaphorase
0,1 - 5 µmol Redoxpartner
1 - 10 µmol Indikatorsubstanz
50 -200 µmol Puffer, pH 6 - 9

Besonders bevorzugt ist ein Testgemisch, das in 2 ml Wasser

5 µmol NAD(P)H
0,5 µmg Diaphorase
1 µmol Redoxpartner
1 µmol Hydroxylamin und
60 - 100 µmol Phosphatpuffer, pH 7,5 - 8,0 enthält

Die selektive Reduktion des Sauerstoffs mit NAD(P)H in einem Zwei-Elektronenschritt zu Wasserstoffperoxid in Gegenwart einer NAD(P)H-abhängigen, nicht-autoxidablen Diaphorase, eines geeigneten Zwei-Elektronen-Redoxpartners sowie gegebenenfalls eines geeigneten Puffersystems ermöglicht auch eine leicht durchzuführende NAD(P)H-Bestimmung. Hierzu wird die zu untersuchende Probe mit Sauerstoff gesättigt und mit einem Testgemisch versetzt, das eine NAD(P)H-abhängige, nicht autoxidable Diaphorase einen geeigneten Zwei-Elektronen-Redoxpartner, gegebenenfalls ein geeignetes Puffersystem sowie ein Wasserstoffperoxid-Nachweissystem enthält. Die mit Hilfe des Wasserstoffperoxid-Nachweissystem gemessene Wasserstoffperoxid-Konzentration ist ein direktes Maß für das in der Probe vorhandene NAD(P)H.

Die Erfindung wird durch die folgenden Beispiele näher erläutert.

## Beispiel 1:

### Erzeugung und Bestimmung von Superoxid

In ein 10 ml Reagenzgefäß werden 2 ml Phosphatpuffer, 0,2 M pH 7,8 gegeben. Es wird 30 Min. bei Raumtemperatur Luft durch·die Pufferlösung hindurchgeleitet, um diese mit Luft zu sättigen. Danach wird das Gemisch mit

4

0,1 ml einer wäßrigen Lösung, die 1 μmol NADH enthält, sowie mit

0,1 ml einer wäßrigen Lösung, die 0,4 μmol Anthrachinon-2-sulfonsäure enthält

versetzt. Die so erhaltene Lösung wird mit 0,5 mg Diaphorase (Boehringer Mannheim, von Mikroorganismen, lyophilisiert), gelöst in 1 ml destiliertes Wasser, vermischt. Das entstehende Superoxid wird mit Hilfe der bekannten Hydroxylamin-Reaktion nachgewiesen.

Hierzu wird dem Reaktionsgemisch zusätzlich zu den bereits genannten Substanzen 0,1 ml einer Hydroxylammoniumchlorid-Lösung, hergestellt durch Lösen von 6,9 mg Hydroxylammoniumchlorid in 10 ml destiliertem Wasser, zugegeben. Die entstehenden Nitrit-Ionen werden durch Zugabe einer 1%igen (w/v) Sulfanilamid-Lösung in 25% Salzsäure und nach Zugabe von 0,02%iger wäßriger Naphthylethylendiamin-Dihydrochlorid sowie durch Messen des gebildeten Azofarbstoffes bei 540 nm bestimmt.

Aus den gemessenen Extinktionswerten läßt sich die Ausbeute an Superoxid durch Vergleich mit einer mit bekannten Nitrit-Konzentrationen ermittelten Eichkurve bestimmen.

Ähnliche Ergebnisse werden erzielt, wenn anstelle der oben erwähnten Anthrachinon-2-sulfonsäure andere Ein-Elektronen-Rezeptoren, wie 1,1'-Dimethyl-4,4'-bipyridiliumchlorid, 1-Methyl-4-(4,6-dimethyl-2-symtriazinyl)-pyridinium-bromid, 1-Methyl-4-(2-symtriazi-nyl)-pyridinium-bromid oder Nitrofurantoin, eingesetzt werden.

### Beispiel 2:

#### Erzeugung von Wasserstoffperoxid

Wie in Beispiel 1 beschrieben, werden in einem 10 ml Reagenzgefäß 2 ml luftgesättigter Phosphatpuffer, 0,2 M, pH 7,8 vorbereitet. Hierzu werden zugegeben.

0,1 ml einer wäßrigen Lösung, die 1 μmol NADH enthält und

0,1 ml einer wäßrigen Lösung, die 0,4 μmol 2,5-Dibromo-3-methyl-6-isopropyl-p-benzochinon enthält.

Die so erhaltene Lösung wird mit 0,5 mg Diaphorase (Boehringer Mannheim, von Mikroorganismen, lyophilisiert), gelöst in 1 ml dest. Wasser, vermischt. Das sich bildende Wasserstoffperoxid wird nach bekannter Methode bestimmt, beispielsweise nach der Methode nach TRINDER.

Vergleichbare Ergebnisse werden erhalten, wenn anstelle des oben erwähnten Redoxpartners andere Zwei-Elektronen-Redoxpartner, beispielsweise 2,3-Dimethyl-5,6-methylendioxy-p-benzochinon, eingesetzt werden.

### Beispiel 3:

#### Erzeugung und Bestimmung von Hydroxyl-Radikalen

A) In ein 10 ml-Reagenzglas werden 2 ml Phosphatpuffer, 0,2 M, pH 7,8 gefüllt. Nacheinander werden unter anaeroben Bedingungen zugesetzt: 0,1 ml einer wäßrigen Lösung, die 0,1 μmol NADH enthält,

0,1 ml einer wäßrigen Lösung, die 0,4 μmol Anthrachinon-2-sulfonsäure enthält,

0,1 ml einer 2%igen Wasserstoffperoxid-Lösung und

0,5 mg Diaphorase (Boehringer Mannheim, von Mikroorganismen, lyophilisiert), in 1 ml dest. Wasser gelöst.

Die entstehenden Hydroxyl-Radikale werden in bekannter Weise mit Hilfe der Methionin-Spaltung nachgewiesen. Aus Methionin wird Ethylen abgespalten, das gaschromatographisch gemessen werden kann.

B) In ein 10 ml-Reagenzglas werden 2 ml Phosphatpuffer, 0,2 M, pH 7,8 gegeben, der, wie in Beispiel 1 beschrieben, mit Luft gesättigt worden ist. Ferner werden zugesetzt:

0,1 ml einer wäßrigen Lösung, die 0,1 μmol NADH enthält,

0,1 ml einer wäßrigen Lösung, die 0,4 μmol N-(5-Nitro-2-furyliden)-1-aminohydantoin (Nitrofurantoin) enthält, 200 Einheiten(definiert in Beispiel 4)Superoxiddismutase

1 mg NADP-Ferredoxin-(Cyt c)-Oxidoreduktase, in 1 ml dest. Wasser gelöst.

Die sich bildenden Hydroxyl-Radikale werden, wie oben beschrieben, durch Zusatz von Methionin bestimmt.

### Beispiel 4:

#### Bestimmung von Superoxiddismutase

#### 1. Erstellen einer Eichkurve

In ein 10 ml Teströhrchen werden 0,5 ml Wasser gegeben und hierzu die folgenden wäßrigen Lösungen Pipettiert:

1,0 ml luftgesättigter Phosphat-Puffer, pH 7,8 (65 μmol)

0,1 ml Hydroxylamin-hydrochlorid (1,0 μmol)

0,1 ml Anthrachinon-2-sulfonsäure (1,0 μmol)

0,1 ml Diaphorase (0,5 mg Protein)

Das Lösungsgemisch wird kräftig geschüttelt und danach mit 0,1 ml Superoxiddismutase (steigende Konzentrationen von 0 - 100 Einheiten und 0,1 ml NADH (5 μmol) versetzt.

Die erhaltene Lösung wird kräftig durchmischt und 15 Min. bei 22°C inkubiert. Nach dieser Reaktionszeit werden 0,5 ml dieser Reaktionslösung mit Nitrit-Reagenz vermischt, das aus

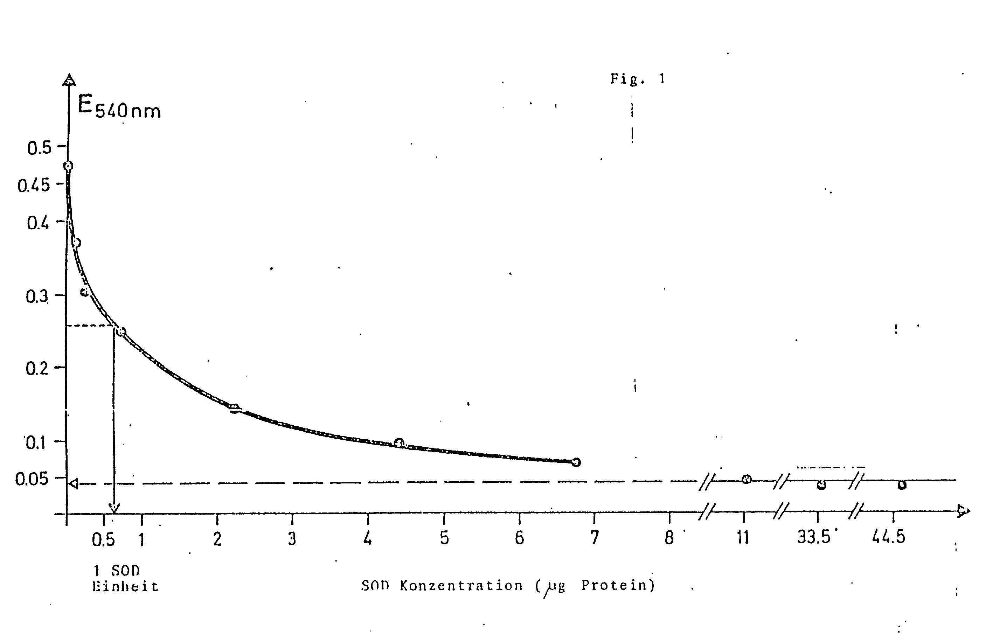

0,5 ml einer 1%igen (w/v) Sulfanilamid-Lösung in 25%iger Salzsäure und
0,5 ml einer 0,02%igen (w/v) Naphthylethylendiamin-dichlorid-Lösung in Wasser
besteht. Die Extinktion bei 540 nm wird spektrophotometrisch verfolgt. In Fig. 1 ist die sich ergebende Eichkurve wiedergegeben.

Für die Superoxiddismutase ist eine Einzymeinheit bestimmt als die Enzymmenge, die eine 50%ige Inhibierung der Detektor-Reaktion, im vorliegenden Fall der Hydroxylamin-Oxidation zu Nitrit-Ionen, bewirkt.

Aus Fig. 1 ergibt sich für das untersuchte Enzym eine Aktivität von einer Einheit pro 0,7 ug Protein nach folgender Gleichung:

$$E\ 1/2 = \frac{E_{max}^{540} + E_{Endwert}^{540}}{2} = \frac{0.475 + 0.04}{2} = 0,26 = 1\ SOD\text{-}Einheit$$

### 2. Bestimmung unbekannter Superoxiddismutase-Konzentrationen

In der gleichen Weise wie vorstehend beschrieben, können auch unbekannte Superoxiddismutase-Konzentrationen bestimmt werden, in dem man anstelle der Standards mit bekannter Superoxiddismutase-Konzentration die entsprechende Menge einer Probe mit unbekanntem Superoxiddismutase-Gehalt einsetzt, die gemessenen Extinktionswerte mit der Eichkurve vergleicht und somit die dem gemessenen Extinktionswert entsprechende Superoxiddismutase-Konzentration bestimmt.

### Beispiel 5:

### Bestimmung von NADH

Wie in Beispiel 2 beschrieben wird Wasserstoffperoxid erzeugt. Es wird lediglich anstelle der Lösung mit bekannten NADH-Gehalt die Probelösung mit unbekannten NADH-Gehalt eingesetzt. Die gebildete Menge an Wasserstoffperoxid wird, nach dem die Reaktion der Wasserstoffperoxid-Bildung aus NADH unter vollständigem Verbrauch von NADH abgelaufen ist, wie folgt bestimmt:

Es werden die folgenden Lösungen zubereitet:

Lösung 1: 100 mg Dichlorphenolsulfansäure werden in 4 ml Ethanol gelöst und mit 4 ml Wasser verdünnt.
Lösung 2: 0,1 g 4-Aminoantipyrin werden in 2,5 ml Wasser gelöst.
Lösung 3: 10 mg Peroxidase werden in 10 ml Wasser gelöst.

Zur Bestimmung des Wasserstoffperoxid werden zu 1 ml der zu testenden Wasserstoffperoxids enthaltenden Lösung 0,8 ml Phosphatpuffer, 0,1 M, pH 7,0, 0,4 ml der Lösung 1, 0,1 ml der Lösung 2 und zum Starten der Reaktion 0,1 ml der Lösung 3 hinzugegeben. Nach 30 Min. Reaktionszeit wird die Extinktion bei 540 nm gemessen. Durch Vergleich des gefundenen Extinktionswertes mit einer entsprechenden Eichkurve wird der NADH-Gehalt der untersuchten Probe bestimmt.

Die Eichkurve wird erhalten, in dem das obige Bestimmungsverfahren mit Proben durchgeführt wird, die verschiedene, jeweils bekannte NADH-Konzentrationen aufweisen.

### Patentansprüche

1. Verfahren zur selektiven Herstellung der reduzierten Sauerstoffspezies Superoxid, Wasserstoffperoxid und Hydroxyl-Radikal, dadurch gekennzeichnet, daß Sauerstoff mit NAD(P)H in Gegenwart einer NAD(P)H-abhängigen, nicht-autoxidablen Diaphorase, eines geeigneten autoxidablen Redoxpartners sowie gegebenenfalls eines geeigneten Puffersystems reduziert wird.

2. Verfahren gemäß Anspruch 1, dadurch gekennzeichnet, daß als NAD(P)H-abhängige, nicht autoxidable Diaphorase NADP-Ferredoxin-(Cyt c)-oxidoreduktase oder Diaphorase aus Clostridium kluyveri eingesetzt wird.

3. Verfahren gemäß einem der Ansprüche 1 und 2, dadurch gekennzeichnet, daß als autoxidable Redoxpartner einen Ein-Elektronenschritt induzierende Substanzen mit einem Ein-Elektronen-Redoxpotential $E_o'$ im Bereich von -150 mV bis -500 mV verwendet werden und Superoxid hergestellt wird.

4. Verfahren gemäß Anspruch 3, dadurch gekennzeichnet, daß als autoxidable Redoxpartner Bipyridilium-Salze, Triazonium-Salze, Anthrachinon-Derivate, Nitrofuran-Derivate, Ferredoxine, Pteridine oder Isoalloxazine eingesetzt werden.

5. Verfahren gemäß einem der Ansprüche 1 und 2, dadurch gekennzeichnet, daß als autoxidable Redoxpartner einen Zwei-Elektronenschritt induzierende Substanzen mit einem Zwei-Elektronen-Redoxpotential $E_o'$ in dem Bereich von 0 mV bis 150 mV verwendet werden und Wasserstoffperoxid hergestellt wird.

6. Verfahren gemäß Anspruch 5, dadurch gekennzeichnet, daß als autoxidable Redoxpartner Chinon-Derivate eingesetzt werden.

7. Verfahren gemäß einem der Ansprüche 1 oder 2, dadurch gekennzeichnet, daß als autoxidable Redoxpartner einen Ein-Elektronenschritt induzierende Substanzen mit einem Ein-Elektronen-Redoxpotential $E'_o$ in dem Bereich von -150 mV bis -500 mV verwendet werden, in Gegenwart von Superoxiddismutase gearbeitet wird und Hydroxyl-Radikale hergestellt werden.

8. Verfahren zur selektiven Herstellung von Hydroxyl-Radikalen, dadurch gekennzeichnet, daß unter anaeroben Bedingungen Wasserstoffperoxid mit NAD(P)H in Gegenwart einer NAD(P)Habhängigen nicht-autoxidablen Diaphorase und eines autoxidablen, einen Ein-Elektronenschritt induzierenden Redoxpartners mit einem Ein-Elektronen-Redoxpotential $E'_o$ in dem Bereich von -150 mV bis -500 mV reduziert wird.

9. Reagenz zur Erzeugung der reduzierten Sauerstoffspezies Superoxid, Wasserstoffperoxid und Hydroxyl-Radikal nach einem Verfahren gemäß einem der Ansprüche 1 bis 8, dadurch gekennzeichnet, daß es NAD(P)H, eine NAD(P)Habhängige, nicht-autoxidable Diaphorase, einen geeigneten autoxidablen Redoxpartner sowie gegebenenfalls ein geeignetes Puffersystem enthält.

10. Reagenz gemäß Anspruch 9, dadurch gekennzeichnet, daß es als NAD(P)H-abhängige, nicht-autoxidable Diaphorase
NADP-Ferredoxin-(Cyt c)-oxidoreductase oder Diaphorase aus Clostridium kluyveri enthält. 11. Reagenz gemäß einem der Ansprüche 9 und 10, dadurch gekennzeichnet, daß es als autoxidablen Redoxpartner eine Substanz, die einen Ein-Elektronenschritt zu induzieren vermag und ein Ein-Elektronen-Redoxpotential $E'_o$ im Bereich von -150 mV bis -500 mV aufweist, enthält und zur Erzeugung von Superoxid dient.

12. Reagenz gemäß Anspruch 11, dadurch gekennzeichnet, daß es als Redoxpartner Bipyridilium-Salze, Triazonium-Salze, Anthrachinon-Derivate, Nitrofuran-Derivate, Ferredoxine, Pteridine oder Isoalloxazine enthält.

13. Reagenz gemäß einem der Ansprüche 9 und 10, dadurch gekennzeichnet, daß es als autoxidablen Redoxpartner eine Substanz, die einen Zwei-Elektronenschritt zu induzieren vermag und ein Zwei-Elektronen-Redoxpotential $E'_o$ im Bereich von 0 mV bis 150 mV aufweist, enthält und zur Erzeugung von Wasserstoffperoxid dient.

14. Reagens gemäß Anspruch 13, dadurch gekennzeichnet, daß es als Redoxpartner Chinon-Derivate enthält.

15. Reagenz gemäß einem der Ansprüche 9 und 10, dadurch gekennzeichnet, daß es als autoxidablen Redoxpartner eine Substanz die einen Ein-Elektronenschritt zu induzieren vermag und ein Ein-Elektronen-Redopotential E' im Bereich von -150 mV bis -500 mV aufweist, zusätzlich Superoxiddismutase enthält und zur Erzeugung von Hydroxyl-Radikalen dient.

16. Verfahren zur Bestimmung von Superoxiddismutase, in dem eine Superoxid-liefernde Reaktion mit zwei konkurrierenden Superoxid-verbrauchenden Reaktionen gekoppelt wird, wobei eine Superoxid-verbrauchende Reaktion eine übliche Indikatorreaktion und die andere Superoxid-verbrauchende Reaktion die Superoxiddismutase-Reaktion darstellt, dadurch gekennzeichnet, daß als Superoxidliefernde Reaktion die Reduktion von Sauerstoff mit NAD(P)H in Gegenwart einer NAD(P)H-abhängigen. nichtautoxidablen Diaphorase, eines autoxidablen, einen Ein-Elektronenschritt induzierenden Redoxpartners mit einem Ein-Elektronen-Redoxpotential im Bereich von -150 mV bis -500 mV und gegebenenfalls eines geeigneten Puffersystems eingesetzt wird.

17. Verfahren gemäß Anspruch 16, dadurch gekennzeichnet, daß als NAD(P)H-abhängige, nicht-autoxidable Diaphorase
NADP-Ferredoxin-(Cyt c)-oxidoreduktase oder Diaphorase aus Clostridium kluyveri eingesetzt wird.

18. Verfahren gemäß einem der Ansprüche 16 und 17, dadurch gekennzeichnet, daß als Redoxpartner Bipyridilium-Salze, Triazonium-Salze, Anthrachinon-Derivate, Nitrofuran-Derivate, Ferredoxine, Pteridine oder Isoalloxazine eingesetzt werden.

19. Reagenz zur Bestimmung der Superoxiddismutase, dadurch gekennzeichnet, daß es NAD(P)H, eine NAD(P)H-abhängige, nicht-autoxidable Diaphorase, einen autoxidablen, einen Ein-Elektronenschritt induzierenden Redoxpartner mit einem Ein-Elektronen-Redoxpotential im Bereich von -150 mV bis -500 mV, die Komponenten für eine geeignete Indikatorreaktion sowie gegebenenfalls ein geeignetes Puffersystem enthält.

20. Reagenz gemäß Anspruch 19, dadurch gekennzeichnet, daß es als NAD(P)H-abhängige, nicht-autoxidable Diaphorase NADP-Ferredoxin-(Cyt c)-oxidoreduktase oder Diaphorase aus Clostridium kluyveri enthält.

21. Reagenz gemäß einem der Ansprüche 19 und 20, dadurch gekennzeichnet, daß es als Redoxpartner Bipyridilium-Salze, Triazonium-Salze, Anthrachinon-Derivate, Nitrofuran-Derivate, Ferredoxine, Pteridine oder Isoalloxazine enthält.

22. Reagenz gemäß einem der Ansprüche 19 bis 21, dadurch gekennzeichnet, daß es als Komponenten für eine Indikatorreaktion ein Hydroxylammonium-Salz und ein Testsystem zum Nachweis von Nitritionen enthält.

23. Verfahren zur Bestimmung von NAD(P)H und NAD(P)H liefernden Reaktionen, indem Sauerstoff durch NAD(P)H zu Wasserstoffperoxid reduziert und letzteres in bekannter Weise bestimmt wird, dadurch gekennzeichnet, daß die Reduktion des Sauerstoffs durch NAD(P)H in Gegenwart einer NAD(P)H-abhängigen, nicht-autoxidablen Diaphorase, eines geeigneten Zwei-Elektronen-Redoxpartners mit einem Zwei-Elektronen-Redoxpotential im Bereich von 0 mV bis 150 mV und eines geeigneten Puffersystems durchgeführt wird.

24. Verfahren gemäß Anspruch 23, dadurch gekennzeichnet, daß als NAD(P)H-abhängige, nicht-autoxidable Diaphorase
NADP-Ferredoxin-(Cyt c)-oxidoreductase oder Diaphorase aus Clostridium kluyveri verwendet wird.

25. Verfahren gemäß einem der Ansprüche 23 und 24, dadurch gekennzeichnet, daß als Redoxpartner Chinon-

7

Derivate verwendet werden.

26. Reagenz zur Bestimmung von NAD(P)H und NAD(P)H liefernden Reaktionen gemäß Anspruch 23, dadurch gekennzeichnet, daß es in Form einer Lösung, eines Pulvergemisches, einer Reagenztablette oder eines Lyophilisates eine NAD(P)H-abhängige, nicht-autoxidable Diaphorase, einen geeigneten Zwei-Elektronen-Redoxpartner mit einem Zwei-Elektronen-Redoxpotential im Bereich von 0 mV bis 150 mV, gegebenenfalls ein geeignetes Puffersystem sowie ein Wasserstoffperoxid-Nachweissystem enthält.

## Revendications

1. Procédé pour la préparation sélective d'oxygène réduit sous lesformes de superoxyde, de peroxyde d'hydrogène et du radical hydroxyle, caractérisé en ce que l'oxygène est réduit avec du NAD(P)H en présence d'une diaphorase non auto-oxydable dépendant du NAD(P)H, d'un partenaire d'oxydo-réduction auto-oxydable approprié ainsi qu'éventuellement d'un système tampon approprié.

2. Procédé selon la revendication 1, caractérisé en ce qu'on utilise comme diaphorase non auto-oxydable dépendant du NAD(P)H, la NADP-ferrédoxine(cyt c)-oxydo-réductase ou la diaphorase provenant du Clostridium kluyveri.

3. Procédé selon l'une des revendications 1 et 2, caractérisé en ce qu'on utilise comme partenaires d'oxydo-réduction auto-oxydables des substances induisant la transfert d'un électron présentant un potentiel d'oxydoréduction $E'_0$ à un électron compris entre -150 et -500mV, pour la préparation de superoxyde.

4. Procédé selon la revendication 3, caractérisé en ce qu'on utilise comme partenaires d'oxydo-réduction auto-oxydables, des sels de dipyridilium, des sels de triazonium, des dérivés d'anthraquinone, des dérivés de nitrofuranne, des ferrédoxines, des ptéridines ou des iso-alloxazines.

5. Procédé selon l'une des revendications 1 et 2, caractérisé en ce qu'on utilise comme partenaires d'oxydo-réduction auto-oxydables des substances induisant un transfert de deux électrons présentant un potentiel $E'_0$ à deux électrons compris entre 0 et 150 mV pour la préparation de peroxyde d'hydrogène.

6. Procédé selon la revendication 5, caractérisé en ce qu'on utilise comme partenaires d'oxydo-réduction auto-oxydables des dérivés de quinone.

7. Procédé selon l'une des revendications 1 et 2, caractérisé en ce qu'on utilise comme partenaires auto-oxydables, des substances induisant un transfert d'un électron présentant un potentiel d'oxydo-réduction à un électron compris entre -150 et -500 mV, et en ce qu'on opère en présence de superoxyde-dismutase, pour la préparation de radicaux hydroxyle.

8. Procédé pour la préparation sélective du radical hydroxyle, caractérisé en ce qu'on réduit dans des conditions anaérobies du peroxyde d'hydrogène avec du NAD(P)H en présence d'une diaphorase non auto-oxydable dépendant du NAD(P)H et d'un partenaire d'oxydo-réduction auto-oxydable induisant un transfert d'un électron présentant un potentiel d'oxydo-réduction à un électron $E'_0$ compris entre -150 mV et -500 mV.

9. Réactif pour l'obtention d'oxygène réduit sous forme de superoxyde, de peroxyde d'hydrogène ou du radical hydroxyle selon le procédé défini dans l'une des revendications 1 à 8, caractérisé en ce qu'il comprend du NAD(P)H, une diaphorase non auto-oxydable dépendant du NAD(P)H, un partenaire d'oxydoréduction auto-oxydable approprié ainsi qu'éventuellement un système tampon approprié.

10. Réactif selon la revendication 9, caractérisé en ce qu'il comprend comme diaphorase non auto-oxydable dépendant du NAD(P)H, de la NADPferrédoxine-(cyt c)-oxydo-réductase, ou de la diaphorase provenant du Clostridium kluyveri.

11. Réactif selon l'une des revendications 9 et 10, caractérisé en ce qu'il comprend comme partenaire d'oxydo-réduction auto-oxydable, une substance susceptible d'induire un transfert d'un électron et présentant un potentiel d'oxydo-réduction à un électron $E_0'$ compris entre -150mV et -500mV, pour la préparation de superoxyde.

12. Réactif selon la revendication 11, caractérisé en ce qu'il contient comme partenaire d'oxydo-réduction un sel de dipyridilium, un sel de triazonium, un dérivé d'anthraquinone, un dérivé de nitrofuranne, de la ferredoxine, de la ptéridine ou de l'iso-alloxazine.

13. Réactif selon l'une des revendications 9 et 10, caractérisé en ce qu'il comprend comme partanaire d'oxydo-réduction auto-oxydable une substance susceptible d'induire un transfert de deux électrons et présentant un potentiel d'oxydo-réduction à deux électrons $E'_0$ compris entre 0et 150 mV, pour l'obtention de peroxyde d'hydrogène.

14. Réactif selon la revendication 13, caractérisé en ce qu'il contient comme partenaire d'oxydo-réduction un dérivé de quinone.

15. Réactif selon l'une des revendications 9 et 10, caractérisé en ce qu'il comprend comme partenaire d'oxydo-réduction auto-oxydable une substance susceptible d'induire un transfert d'un électron et présentant un potentiel d'oxydo-réduction à un électron $E'_0$ compris entre -150 mVet -500 mV, et en ce qu'il comprend en outre de la superoxyde-dismutase, pour l'obtention de radicaux hydroxyle.

16. Procédé pour le dosage de superoxyde-dismutase, dans lequel une réaction fournissant du superoxyde est couplée à deux réactions concurrentes consommatrices de superoxyde, l'une des réactions consommatrices de superoxyde étant une réaction indicatrice habituelle et l'autre réaction consommatrice de superoxyde étant la réaction de superoxyde-dismutase, caractérisé en ce qu'on utilise comme réaction fournissant du superoxyde, la réduction d'oxygène avec du NAD(P)H en présence d'une diaphorase non auto-oxydable dépendant du NAD(P)H, d'un partenaire d'oxydo-réduction auto-oxydable induisant un transfert d'un électron présentant un potentiel d'oxydo-réduction à un électron compris entre -150 mV et -500 mV et éventuellement d'un système tampon approprié.

17. Procédé selon la revendication 16, caractérisé en ce qu'on utilise comme diaphorase non auto-oxydable dépendant du NAD(P)H, la NADP-ferrédoxine-(cyt c)-oxydo-réductase ou la diaphorase provenant du Clostridium kluyveri.

18. Procédé selon l'une des revendications 16 et 17, caractérisé en ce qu'on utilise comme partenaires d'oxydo-réduction des sels de dipyridilium, des sels de triazonium, des dérivés d'anthraquinone, des dérivés de nitrofuranne, des ferrédoxines, des ptéridines ou des iso-alloxazines.

19. Réactif pour le dosage de la superoxyde-dismutase, caractérisé en ce qu'il comprend du NAD(P)H, une diaphorase non auto-oxydable dépendant du NAD(P)H, un partenaire d'oxydo-réduction auto-oxydable induisant un transfert d'un électron présentant un potentiel d'oxydo-réduction à un électron compris entra -150 mV et -500 mV, les composants permettant une réaction indicatrice appropriée ainsi qu'éventuellement un système tampon approprié.

20. Réactif selon la revendication 19, caractérisé en ce qu'il contient comme diaphorase non auto-oxydable dépendant du NAD(P)H, la NADP-ferrédoxine-(cyt c)-oxydo-réductase ou la diaphorase provenant du Clostridium kluyveri.

21. Réactif selon l'une des revendications 19 et 20, caractérisé en ce qu'il comprend comme partenaire d'oxydo-réduction un sel de dipyridilium, un sel de triazonium, un dérivé d'anthraquinone, un dérivé de nitrofuranne, des ferrédoxines, des ptéridines ou des iso-alloxazines.

22. Réactif selon l'une des revendications 19 à 21, caractérisé en ca qu'il contient comme composants pour une réaction indicatrice un sel d'hydroxylammonium et un système d'essai pour la mise en évidence d'ions nitrite.

23. Procédé pour la dosage de NAD(P)H et de réactions donnant du NAD(P)H, en soumettant de l'oxygène à une réduction par du NAD(P)H pour le transformer en peroxyde d'hydrogène, ca dernier étant dosé de la façon habituelle, caractérisé an ce que la réduction de l'oxygène par le NAD(P)H est réalisée en présence d'une diaphorase non auto-oxydable dépendant du NAD(P)H, d'un partenaire d'oxydo-réduction approprié à deux électrons présentant un potentiel d'oxydo-réduction à deux électrons compris entre 0 et 150 mV et d'un système tampon approprié.

24. Procédé salon la revendication 23, caractérisé an ca qu'on utilise comme diaphorase non auto-oxydable dépendant du NAD(P)H, la NADP-ferrédoxine-(cyt c)-oxydo-réductase ou la diaphorase provenant du Clostridium kluyveri.

25. Procédé selon l'une das revendications 23 at 24, caractérisé en ca qu'on utilise comme partenaire d'oxydo-réduction des dérivés de quinone.

26. Réactif pour le dosage de NAD(P)H et de réactions fournissant du NAD(P)H selon la revendication 23, caractérisé en ce qu'il comporte, sous la forme d'une solution, d'un mélange pulvérulent, d'un comprimé réactif ou d'un lyophilisat, une diaphorase non auto-oxydable dépendant du NAD(P)H, un partenaire d'oxydo-réduction à deux électrons ayant un potentiel d'oxydo-réduction à deux électrons compris entre 0 et 150 mV, éventuellement un système tampon approprié ainsi qu'un système pour la mise en évidencede peroxyde d'hydrogène.

## Claims

1. Process for the selective production of the reduced oxygen species superoxide, hydrogen peroxide and hydroxyl radicals, characterised in that oxygen is reduced with NAD(P)H in the presence of an NAD(P)Hdependent, non-autoxidisable diaphorase, of a suitable autoxidisable redox partner, as well as possibly of a suitable buffer system.

2. Process according to claim 1, characterised in that, as NAD(P)H-dependent, non-autoxidisable diaphorase, there is used NADP-ferredoxin-(Cyt c)-oxidoreductase or diaphorase from Clostridium kluyveri.

3. Process according to one of claims 1 and 2, characterised in that, as autoxidisable redox partner, there is used a one-electron step-inducing substance with a one-electron redox potential $E'_0$ in the range of -150 mV to -500 mV and superoxide is produced.

4. Process according to claim 3, characterised in that, as autoxidisable redox partners, there are used bipyridilium salts, triazonium salts, anthraquinone derivatives, nitrofuran derivatives, ferredoxins, pteridines or isoalloxazines.

5. Process according to one of claims 1 and 2, characterised in that, as autoxidisable redox partners, there are used two-electron step-inducing substances with a two-electron redox potential $E_0'$ in the range of 0 mV to 150 mV and hydrogen peroxide is produced.

6. Process according to claim 5, characterised in that, as autoxidisable redox partners, there are used quinone derivatives.

7. Process according to one of claims 1 and 2, characterised in that, as autoxidisable redox partners, there are used one-electron step-inducing substances with a one-electron redox potential $E'_0$ in the range of -150 mV to -500 mV in the presence of superoxide dismutase and hydroxyl radicals are produced.

8. Process for the selective production of hydroxyl radicals, characterised in that, under anaerobic conditions, hydrogen peroxide is reduced with NAD(P)H in the presence of an NAD(P)H-dependent, nonautoxidisable diaphorase and of an autoxidisable, one-electron step-inducing redox partner with a one-electron redox potential $E'_0$ in the range of -150 mV to -500 mV.

9. Reagent for the production of the reduced oxygen species superoxide, hydrogen peroxide and hydroxyl radicals according to a process according to one of claims 1 to 8, characterised in that it contains NAD(P)H, an NAD(P)H-dependent, non-autoxidisable diaphorase, an appropriate autoxidisable redox partner, as well as possibly a suitable buffer system.

10. Reagent according to claim 9, characterised in that, as NAD(P)H-dependent, non-autoxidisable diaphorase, it contains NADP-ferredoxin-(Cyt c)oxidoreductase or diaphorase from <u>Clostridium kluyveri</u>.

11. Reagent according to one of claims 9 and 10, characterised in that, as autoxidisable redox partner, it contains a substance which is able to induce a one-electron step and has a one-electron redox potential $E'_0$in the range of -150 mV to -500 mV and serves for the production of superoxide.

12. Reagent according to claim 11, characterised in that, as redox partner, it contains bipyridilium salts, triazonium salts, anthraquinone derivatives, nitrofuran derivatives, ferredoxins, pteridines or isoalloxazines.

13. Reagent according to one of claims 9 and 10, characterised in that, as autoxidisable redox partner, it contains a substance which is able to induce a two-electron step and has a two-electron redox potential $E'_0$ in the range of 0 mV to 150 mV and serves for the production of hydrogen peroxide.

14. Reagent according to claim 13, characterised in that it contains quinone derivatives as redox partners.

15. Reagent according to one of claims 9 and 10, characterised in that, as autoxidisable redox partner, it contains a substance which is able to induce a one-electron step and has a one-electron redox potential $E'_0$ in the range of -150 mV to -500 mV, additionally contains superoxide dismutase and serves for the production of hydroxyl radicals.

16. Process for the determination of superoxide dismutase in which a superoxide-yielding reaction is coupled with two competing superoxide-consuming reactions, whereby one superoxide-consuming reaction represents a conventional indicator reaction and the other superoxide-consuming reaction the superoxide dismutase reaction, characterised in that, as superoxide-yielding reaction, there is used the reduction of oxygen with NAD(P)H in the presence of an NAD(P)H-dependent, non-autoxidisable diaphorase, of an autoxidisable one-electron step-inducing redox partner with a one-electron redox potential in the range of -150 mV to -500 mV and possibly a suitable buffer system.

17. Process according to claim 16, characterised in that, as NAD(P)H-dependent, non-autoxidisable diaphorase, there is used NADP-ferredoxin-(Cyt c)oxidoreductase or diaphorase from <u>Clostridium kluyveri</u>.

18. Process according to one of claims 16 and 17, characterised in that, as redox partners, there are used bipyridilium salts, triazonium salts, anthraquinone derivatives, nitrofuran derivatives, ferredoxins, pteridines or isoalloxazines.

19. Reagent for the determination of superoxide dismutase, characterised in that it contains NAD(P)H, an NAD(P)H-dependent, non-autoxidisable diaphorase, an autoxidisable, one-electron step-inducing redox partner with a one-electron redox potential in the range of -150 mV to -500 mV, the components for a suitable indicator reaction, as well as possibly a suitable buffer system.

20. Reagent according to claim 19, characterised in that, as NAD(P)H-dependent, non-autoxidisable diaphorase, it contains NADP-ferredoxin-(Cyt c)oxidoreductase or diaphorase from <u>Clostridium kluyveri</u>.

21. Reagent according to one of claims 19 and 20, characterised in that, as redox partners, it contains bipyridilium salts, triazonium salts, anthraquinone derivatives, nitrofuran derivatives, ferredoxins, pteridines or isoalloxazines.

22. Reagent according to one of claims 19 to 21, characterised in that, as components for an indicator reaction, it contains a hydroxylammonium salt and a test system for the detection of nitrite ions.

23. Process for the determination of NAD(P)H and NAD(P)H-yielding reactions in which oxygen is reduced by NAD(P)H to hydrogen peroxide and the latter is determined in known manner, characterised in that the reduction of the oxygen by NAD(P)H is carried out in the presence of an NAD(P)H-dependent, non-autoxidisable diaphorase, of a suitable two-electron redox partner with a two-electron redox potential in the range of 0 mV to 150 mV and of a suitable buffer system.

24. Process according to claim 23, characterised in that, as NAD(P)H-dependent, non-autoxidisable diaphorase, there is used NADP-ferredoxin-(Cyt c)-oxidoreductase or diaphorase from <u>Clostridium kluyveri</u>.

25. Process according to one of claims 23 and 24, characterised in that quinone derivatives are used as redox partners.

26. Reagent for the determination of NAD(P)H and NAD(P)H-yielding reactions according to claim 23, characterised in that it is in the form of a solution, of a powder mixture, of a reagent tablet or of a lyophilisate and contains an NAD(P)H-dependent, non-autoxidisable diaphorase, a suitable two-electron redox partner with a two-electron redox potential in the range of 0 mV to 150 mV, possibly a suitable buffer, as well as a hydrogen peroxide detection system.

Fig. 1

E$_{540}$nm

SOD Konzentration ($\mu$g Protein)

1 SOD
Einheit

0 105 443